# EUROPEAN PATENT SPECIFICATION

(11) **EP 2 814 413 B1**
(45) Date of publication and mention of the grant of the patent: **03.05.2017**
(21) Application number: 13749876.2
(22) Date of filing: 04.02.2013
(51) Int. Cl.: A61B 17/86, A61B 17/70, A61L 27/54

(54) **BONE FASTENER AND METHODS OF USE**
KNOCHENFIXIERVORRICHTUNG UND VERWENDUNGSVERFAHREN
ÉLÉMENT DE FIXATION D'OS ET SES PROCÉDÉS D'UTILISATION

(30) Priority: 15.02.2012 US 201213397316
(43) Date of publication of application: 24.12.2014
(73) Proprietor: Warsaw Orthopedic, Inc., Warsaw, IN 46581 (US)
(72) Inventor: BALLARD, Rodney, Lakeland Tennessee 38022 (US); GIL, Carlos, Memphis Tennessee 38104-3905 (US)
(74) Representative: Viering, Jentschura & Partner mbB Patent- und Rechtsanwälte
(86) International application number: PCT/US2013/024589
(87) International publication number: WO 2013/122765

(56) References cited:
- US-A1- 2001 021 852
- US-A1- 2004 225 292
- US-A1- 2007 270 858
- US-A1- 2011 213 426
- US-A1- 2011 213 426
- US-A1- 2012 029 578
- US-B1- 6 214 012
- US-B1- 6 517 542

## Description

### TECHNICAL FIELD

The present disclosure generally relates to medical devices for the treatment of bone disorders, and more particularly to a fenestrated bone fastener that is coated with a bone conductive material on the surface, as well as, in cannulated surfaces in the bone fastener in order to facilitate bone in growth and prevent fastener failure.

### BACKGROUND

Spinal disorders such as degenerative disc disease, disc herniation, osteoporosis, spondylolisthesis, stenosis, scoliosis and other curvature abnormalities, kyphosis, tumor, and fracture may result from factors including trauma, disease and degenerative conditions caused by injury and aging. Spinal disorders typically result in symptoms including pain, nerve damage, and partial or complete loss of mobility.

Non-surgical treatments, such as medication, rehabilitation and exercise can be effective, however, may fail to relieve the symptoms associated with these disorders. Surgical treatments of these spinal disorders include discectomy, laminectomy, fusion and implantable prosthetics. As part of these surgical treatments, spinal constructs such as vertebral rods and bone fasteners are often used to provide stability to a treated region. Rods redirect stresses away from a damaged or defective region while healing takes place so as to restore proper alignment and generally support the vertebral members. Bone fasteners are used to anchor the rods in place and it is imperative that the bone fastener be securely anchored to the bone in order for the other devices, such as rods, to work properly. That is, during surgical treatment, one or more rods may be attached via fasteners to the exterior of two or more vertebral members. This disclosure describes an improved fenestrated bone fastener that provides for improved anchorage in bone over the prior art technologies.

Fenestrated bone fasteners are known from US 2011/213426 A1, US 6 517 542 B1, and US 2007/270858 A1.

### SUMMARY

According to the present invention, a fenestrated bone fastener according to claim 1 is provided. Examples of fenestrated bone fasteners are defined in the dependent claims.

A fenestrated bone fastener that is coated with a bone conductive material on the surface of the fastener, as well as, cannulated surfaces is disclosed which facilitates bone in growth and prevents fastener failure.

In one particular embodiment, in accordance with the principles of the present disclosure, a bone fenestrated fastener is provided. The bone fastener comprises a head and elongated shank defining a longitudinal axis having a proximal end and a distal end. The proximal end is connected to a head and the elongated shank comprises an inner surface defining a longitudinal cavity disposed therein and an outer surface defining the elongated shank. The inner surface further defines at least one transverse cavity positioned in the elongated shank that is in communication with the longitudinal cavity wherein at least the inner surface defining the transverse cavity is sprayed, layered, fused, coated or textured in a manner or with material that facilitates the growth and attachment of bone so as to promote bone growth into and about the transverse cavity.

In one embodiment, in accordance with the principles of the present disclosure, a fenestrated bone fastener having an inner surface defining a longitudinal cavity and the inner surface of the at least one transverse cavity and the longitudinal cavity are sprayed, layered, fused, coated or textured in a manner or with material that facilitates the growth and attachment of bone so as to promote bone growth so as to promote bone growth into and about the longitudinal and the traverse cavities to further secure the bone fastener to bone is provided.

In another embodiment, in accordance with the principles of the present disclosure, a fenestrated bone fastener having an inner surface defining a longitudinal cavity and at least one transverse cavity positioned in the elongated shank of the bone fastener is provided. The fenestrated bone fastener also has an outer surface that defines the shape of the elongated shank wherein the outer and inner surfaces of the bone fastener are sprayed, layered, fused, coated or textured in a manner or with material that facilitates the growth and attachment of bone so as to promote bone growth.

In one embodiment, in accordance with the principles of the present disclosure a fenestrated bone fastener comprising a head and an elongated shank defining a longitudinal axis having a proximal end and a distal end wherein the proximal end is connected to the head. The elongated shank comprises an inner surface defining a longitudinal cavity disposed along the longitudinal axis and at least one transverse cavity positioned in the elongated shank wherein the at least one transverse cavity is in communication with the longitudinal cavity. Either one or all of inner surfaces defining the transverse cavity and the elongated cavity as well as the outer surface of the elongated shank is sprayed, layered, fused, coated or textured in a manner or with material that facilitates the growth and attachment of bone so as to promote bone growth into the transverse cavity, elongated cavity and/or on the surface of the elongated shank in order to further secure the bone fastener to bone. The distal end of the fenestrated bone fastener of the present disclosure is configured to cut and deposit bone fragments into the elongated cavity of the bone fastener as the fastener is inserted into bone so as to self-pack the elongated cavity with autograft material.

### BRIEF DESCRIPTION OF THE DRAWINGS

The present disclosure will become more readily apparent from the specific description accompanied by the following drawings, in which:
FIG. 1 is a side view of one particular embodiment of a bone fastener in accordance with the principles of the present disclosure;
FIG. 1A is an enlarged, detail side view in of a portion of the bone fastener shown in FIG. 1;
FIG. 2 is a isometric view of one particular embodiment of the bone fastener shown in FIG. 1, in accordance with the principles of the present disclosure;
FIG. 3 is a side view of one particular embodiment of the bone fastener in accordance with the principles of the present disclosure;
FIG. 3A is an enlarged, detailed side view in of the bone fastener shown in FIG. 3; and
FIG. 4 is a partial cross-section of the one particular embodiment of the bone fastener in accordance with the principles of the present disclosure showing the cutting features of the distal end.

Like reference numerals indicate similar parts throughout the figures.

### DETAILED DESCRIPTION OF THE INVENTION

The exemplary embodiments of the bone fastener and methods of use disclosed are discussed in terms of medical devices for the treatment of bone disorders and more particularly, in terms of a fenestrated bone fastener having a elongated cannulated core sprayed, layered, fused, coated or textured in a manner or with material that facilitates the growth and attachment of bone so as to promote bone growth to facilitate bone growth inside the elongated cavity as well as outside of the bone fastener for better and early bone digitations to the bone fastener shank. This improves the initial stability of the bone fastener in dynamic fixation devices. It is envisioned that employment of the coated bone fastener of the present disclosure can be used with other implants, for example, such as a vertebral rod system that provides stability in dynamic fixation devices. The coated bone fastener may also be used with other constructs such as plates.

It is envisioned that the coated fenestrated bone fastener of the present disclosure may be employed to treat spinal disorders such as, for example, degenerative disc disease, disc herniation, osteoporosis, spondylolisthesis, stenosis, scoliosis and other curvature abnormalities, kyphosis, tumor and fractures. It is further envisioned that the present disclosure may be employed with surgical treatments including open surgery and minimally invasive procedures, of such disorders, such as, for example, discectomy, laminectomy, fusion, bone graft, implantable prosthetics and/or dynamic stabilization applications. It is contemplated that the present disclosure may be employed with other osteal and bone related applications, including those associated with diagnostics and therapeutics. It is further contemplated that the disclosed coated fenestrated bone fastener may be employed in a surgical treatment with a patient in a prone or supine position, employing a posterior, lateral or anterior approach. The present disclosure may be employed with procedures for treating the lumbar, cervical, thoracic and pelvic regions of a spinal column.

The present invention may be understood more readily by reference to the following detailed description of the invention taken in connection with the accompanying drawing figures, which form a part of this disclosure. It is to be understood that this invention is not limited to the specific devices, methods, conditions or parameters described and/or shown herein, and that the terminology used herein is for the purpose of describing particular embodiments by way of example only and is not intended to be limiting of the claimed invention. Also, as used in the specification and including the appended claims, the singular forms "a," "an," and "the" include the plural, and reference to a particular numerical value includes at least that particular value, unless the context clearly dictates otherwise. Ranges may be expressed herein as from "about" or "approximately" one particular value and/or to "about" or "approximately" another particular value. When such a range is expressed, another embodiment includes from the one particular value and/or to the other particular value. Similarly, when values are expressed as approximations, by use of the antecedent "about," it will be understood that the particular value forms another embodiment.

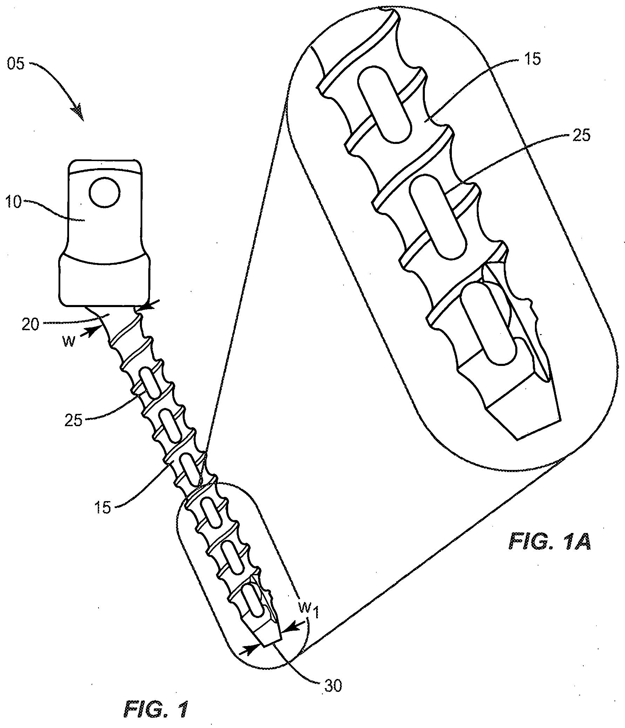

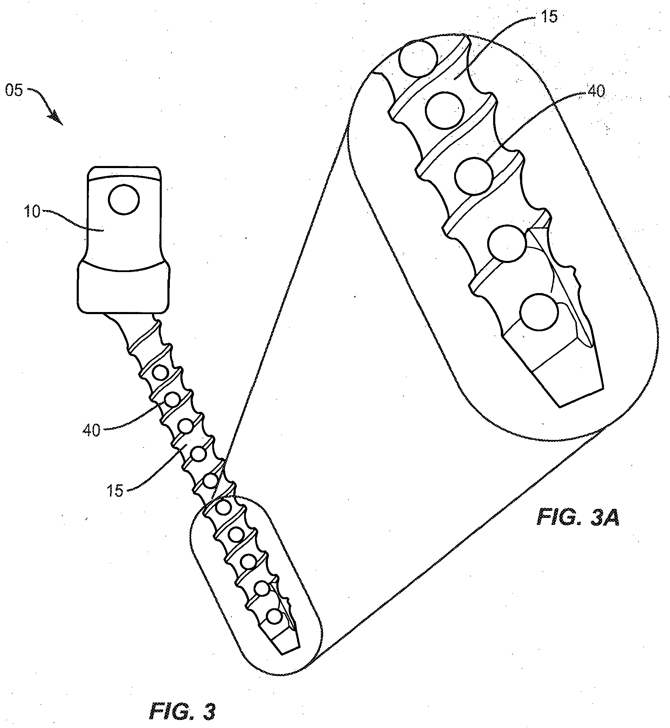

The following discussion includes a description of a coated fenestrated bone fastener, related components and exemplary methods of employing the bone fastener in accordance with the principles of the present disclosure. Alternate embodiments are also disclosed. Reference will now be made in detail to the exemplary embodiments of the present disclosure, which are illustrated in the accompanying figures. Turning now to FIGS. 1-4, there is illustrated components of a fenestrated bone fastener 05 in accordance with the principles of the present disclosure.

Bone fastener 05 comprises an elongated first section, such as, for example, an elongated shank 15 having an outer surface, a proximal end 20 and a distal end 30. Shank 15 has a cylindrical configuration and is configured with at least one transverse cavity 25 positioned in the shank 15. The transverse cavity 25 extends either partially or completely through the shank 15. The traverse cavity 25 can have different configurations, for example, such as spherical, oval, rectangular, triangular, elliptical, and polygonal. In addition, the traverse cavity 25 can also have different spatial orientations, for example, such as angular, off-centered to one another, evenly spaced apart form one another, and unevenly spaced apart from one another. Other configurations are also contemplated to fall within the scope of the present invention.

The proximal end 20 of shank 15 is connected to a head 10 which can be used to drive the bone fastener into bone or can be configured to support a bone construct, such as, for example, vertebral rod. The head 10 can be made from the same or different material than the shank 15 and can be either connected to the shank 15 or formed as a monolithic unit. The head 10 can be attached to the shank 15 so that it can rotate, is angled, is positionable, or fixed. The head 10 can also have different configurations depending on the particular use of the fenestrated bone fastener 05. That is, it can be u-shaped when it is used in connection with a rod system or substantially flat when used with a plate.

Shank 15 has a body that extends from proximal end 20 to distal end 30. The body of the shank 15 can be uniform in width or can taper from a first thickness having a diameter, for example *w* to a second, reduced thickness having a diameter *w₁*. It is contemplated that shank 15 or only portions thereof can have variously dimensioned, for example, with regard to length, width, diameter and thickness. It is further contemplated that in additional to a cylindrical cross-sectional geometry, the shank 15 or only portions thereof, can have various configurations, for example, round, oval, rectangular, irregular, consistent, variable, uniform and non-uniform.

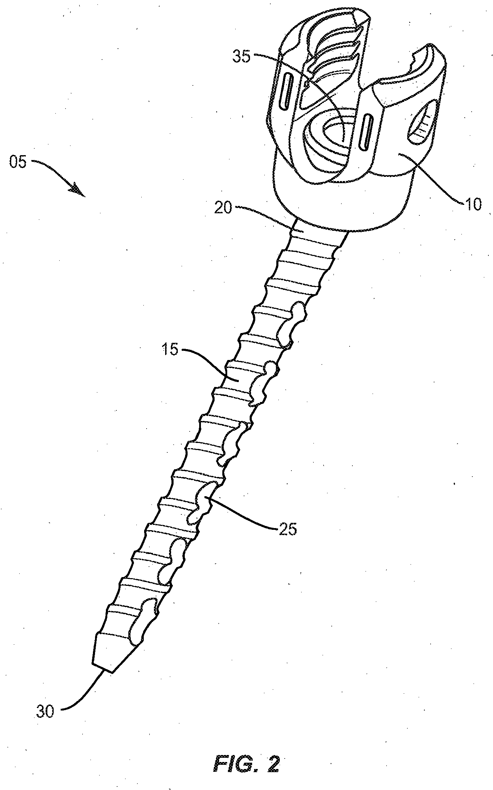

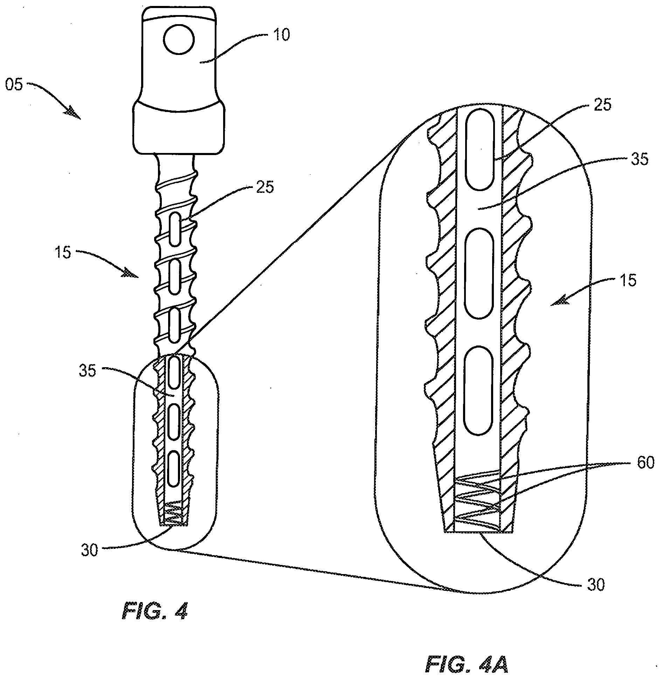

Shank 15 can have an elongated cavity 35, such as a cannulated configuration, as shown in Figures 2 and 4, extending partially or continuously from the proximal end 20 to the distal end 30 of shank 15. The longitudinal cavity 35 formed from the inner surface is in communication with the inner surface defining at least one transverse cavity positioned in the shank 15. In one embodiment of the present disclosure, the inner surface defining the longitudinal cavity 35 and of the traverse cavities 25 are in communication with one another. The inner surface can be continuous, non-interrupted, smooth, textured, or have a porous configuration and is disposed in close fitting engagement with the outer surface that forms the shape and contour of the shank 15. Therefore, in one embodiment of the present disclosure, the outer and inner surfaces being in close engagement with each other makes the traverse cavity 25, elongated cavity 35, and outer surface continuous with one another.

It is contemplated that walls of bone fastener 05 can be variously dimensioned, for example, with regard to the length or thickness of wall 45, and cross sectional geometry such as those discussed above. For example, the cross-sectional geometries of the walls of the bone fastener defining the outer surface and/or inner surface can be round, oval, rectangular, irregular, consistent, variable, uniform and non-uniform, and the inner and outer surfaces may have the same or different cross section geometry. That is, the outer and inner surfaces can be concave, convex, flat, arcuate, multifaceted, or a combination thereof.

Bone fastener 05 may be employed as a bone screw, pedicle screw or multi-axial screw used in spinal surgery. It is contemplated that the all or part of the outer surface, the inner surface defining the traverse cavity 25 or the elongated cavity 35 of the bone fastener 05 may be coated, sprayed, fused, or textured with an osteoconductive and/or osteoinductive material in a manner or with material that facilitates the growth and attachment of bone. The osteoconductive and/or osteoinductive material used is selected from the group consisting of is selected from the group consisting of bone graft, therapeutic polynucleotides or polypeptides, rigid polymers, biocompatible metals, such as titanium elements, metal powders of titanium or titanium compositions, sterile bone materials, such as allograft or xenograft materials, synthetic bone materials such as coral and calcium compositions, such as hyaluronic acid (HA), calcium phosphate and calcium sulfite, biologically active agents, such as, Bone Morphogenetic Proteins (BMP), Growth and Differentiation Factors Proteins (GDF), and cytokines, allogenic demineralized bone, synthetic polymers and copolymers, synthetic copolymers of polyglicolic and poly lactic acid, purified collagen, epidermal growth factor (EGF) platelet derived growth factor (PDGF), fibroblast growth factors (FGFs), parathyroid hormone related peptide (PTHrp), insulin-like growth factors (IGFs) and transforming growth factor-beta (TGF-B).

In one embodiment, in accordance with the principles of the present disclosure, the inner surface of the bone fastener 05 defining the elongated cavity 35 and the traverse cavity 25 is coated, sprayed, fused, or textured with an osteoconductive and/or osteoinductive material and the outer surface of the bone fastener is not. This directs growth into the fenestrations and the elongated cavity of the fenestrated bone fastener that may be beneficial depending on where the bone fastener is being used. The inner surface of the bone fastener 05 defining the elongated cavity 35 and the traverse cavity 25 can be coated, sprayed, fused, or textured with the same, different or a combination of materials with osteoconductive and/or osteoinductive material selected from the group consisting of is selected from the group consisting of bone graft, therapeutic polynucleotides or polypeptides, rigid polymers, biocompatible metals, such as titanium elements, metal powders of titanium or titanium compositions, sterile bone materials, such as allograft or xenograft materials, synthetic bone materials such as coral and calcium compositions, such as hyaluronic acid (HA), calcium phosphate, calcium sulfite, biologically active agents, such as, Bone Morphogenetic Proteins (BMP), Growth and Differentiation Factors Proteins (GDF), cytokines, allogenic demineralized bone, synthetic polymers and copolymers, synthetic copolymers of polyglicolic and polylactic acid, purified collagen, epidermal growth factor (EGF) platelet derived growth factor (PDGF), fibroblast growth factors (FGFs), parathyroid hormone related peptide (PTHrp), insulin-like growth factors (IGFs) and transforming growth factor-beta (TGF-B).

In another embodiment, in accordance with the principles of the present disclosure, the outer surface of the shank 15 along with the inner surface of the bone fastener 05 defining the traverse cavity 25 and not the elongated cavity 35 can be coated, sprayed, fused, or textured with an osteoconductive and/or osteoinductive material in a manner or with material that facilitates the growth and attachment of bone, for example with an osteoconductive and/or osteoinductive material selected from the group consisting of is selected from the group consisting of bone graft, therapeutic polynucleotides or polypeptides, rigid polymers, biocompatible metals, such as titanium elements, metal powders of titanium or titanium compositions, sterile bone materials, such as allograft or xenograft materials, synthetic bone materials such as coral and calcium compositions, such as hyaluronic acid (HA), calcium phosphate and calcium sulfite, biologically active agents, such as, Bone Morphogenetic Proteins (BMP), Growth and Differentiation Factors Proteins (GDF), and cytokines, allogenic demineralized bone, synthetic polymers and copolymers, synthetic copolymers of polyglicolic and polylactic acid, purified collagen, epidermal growth factor (EGF) platelet derived growth factor (PDGF), fibroblast growth factors (FGFs), parathyroid hormone related peptide (PTHrp), insulin-like growth factors (IGFs) and transforming growth factor-beta (TGF-B).

In yet another embodiment, in accordance with the principles of the present disclosure, the outer surface of the shank 15 along with the inner surface of the bone fastener 05 defining elongated cavity 35 and not traverse cavity 25 are all coated, sprayed, fused, or textured with an osteoconductive and/or osteoinductive material in a manner that facilitates the growth and attachment of bone, for example with an osteoconductive and/or osteoinductive material selected from the group consisting of is selected from the group consisting of bone graft, therapeutic polynucleotides or polypeptides, rigid polymers, biocompatible metals, such as titanium elements, metal powders of titanium or titanium compositions, sterile bone materials, such as allograft or xenograft materials, synthetic bone materials such as coral and calcium compositions, such as hyaluronic acid (HA), calcium phosphate, calcium sulfite, biologically active agents, such as, Bone Morphogenetic Proteins (BMP), Growth and Differentiation Factors Proteins (GDF), cytokines, allogenic demineralized bone, synthetic polymers and copolymers, synthetic copolymers of polyglicolic and polylactic acid, purified collagen, epidermal growth factor (EGF) platelet derived growth factor (PDGF), fibroblast growth factors (FGFs), parathyroid hormone related peptide (PTHrp), insulin-like growth factors (IGFs) and transforming growth factor-beta (TGF-B).

In one particular embodiment, in accordance with the principles of the present disclosure any one of or all of the outer surface, the inner surface defining the traverse cavity 25 and the inner surface defining the elongated cavity 35 of the bone fastener 05 is coated with hyaluronic acid (HA) and/or Bone Morphogenetic Proteins (BMP). The outer surface can be coated, sprayed, fused, or textured with one particular osteoconductive and/or osteoinductive material while the inner surface defining the elongated cavity 35 and the transverse cavity 25 is coated, sprayed, fused, or textured with yet a different osteoconductive and/or osteoinductive material. For example, hyaluronic acid (HA) can be used on the outer surface and Bone Morphogenetic Proteins (BMP) can be used to coat the inner surfaces of the elongated cavity 35 and/or the transverse cavity 25.

The outer and inner surfaces can be partially coated or completely coated in order to enhance bony fixation. The outer surface can also be textured so as to enhance bone growth about the bone fastener. As mentioned above, the same osteoconductive and/or osteoinductive materials can be used to coat each surface or in the alternative, a mixture of at least two different osteoconductive and/or osteoinductive materials can be used to coat all or part of the inner and outer surfaces. Bone mineral matrix, allograft, autograft and other materials that facilitate initial bone growth can be inserted inside the elongated and/or transverse cavities 25, 35 of the bone fastener just before implantation in order to provide better initial bone growth within the elongated and/or traverse cavities of the elongated shank 15.

In one embodiment, in accordance with the principles of the present disclosure bone fastener 05 can be pre-packed with Bone mineral matrix, allograft, autograft and/or other materials that facilitate initial bone growth. For example, in one embodiment of the present disclosure, autograft material is loaded inside the elongated cavity 35 and/or the traverse cavity 25. This pre-packing feature allows the bone fastener 05 to be coated, sprayed, fused, or textured with an osteoconductive and/or osteoinductive material in a manner or with material that facilitates the growth and attachment and to be loaded with Bone Matrix material so as to improve biological fixation of the bone fastener when used.

In yet another embodiment of the present disclosure, the bone fastener can be designed to automatically pack the elongated cavity 35 and/or transverse cavity 25 with allograft material that is generated as it is inserted into bone. In this embodiment, distal end 30 of bone fastener 05 comprises cutting features that are configured to generate bone fragments that pack the longitudinal cavity 35 as the bone fastener 05 is driven into bone. As shown in Fig. 4, distal end 30 of the bone fastener has a plurality of blades 60 disposed along the inner surface defining the elongated cavity 35. These blades 60 are positioned at the distal end 30 so as to cut into bone and to self-pack the elongated cavity 35 with autograft material as it is inserted into bone. That is, the cutting blades 60 located at the distal end 30 are configured to have a cutting edge that spirals along at least a portion of the inner surface of the elongated cavity 35 at the distal end so as to shave fragments of bone and direct the fragments into the elongated cavity 35 to fill the elongated cavity 35 with allograft material as the bone fastener is inserted into the bone. This feature is provided in addition to the inner surface defining the elongated cavity 35, transverse cavity 25 and the outer surface of the shank 15 being coated, sprayed, fused, or textured with an osteoconductive and/or osteoinductive material in a manner that facilitates the growth and attachment of bone as discussed above.

The components of bone fastener 05 are fabricated from materials suitable for medical applications, including metals, polymers, ceramics, biocompatible materials and/or their composites, depending on the particular application and/or preference of a medical practitioner. For example, bone fastener 05, discussed below, can be fabricated from materials such as commercially pure titanium, titanium alloys, Grade 5 titanium, super-elastic titanium alloys, cobalt-chrome alloys, stainless steel alloys, superelastic metallic alloys (e.g. Nitinol, super elasto-plastic metals, such as GUM METAL® manufactured by Toyota Material Incorporated of Japan), thermoplastics such as polyaryletherketone (PAEK) including polyetheretherketone (PEEK), polyetherketoneketone (PEKK) and polyetherketone (PEK), carbon fiber reinforced PEEK composites, PEEK-BaSO₄ composites, ceramics and composites thereof such as calcium phosphate (e.g. SKELITE^{™} manufactured by Biologix Inc.), rigid polymers including polyphenylene, polyamide, polyimide, polyetherimide, polyethylene, polyurethanes of any durometer, epoxy and silicone. Different components of the bone fastener may have alternative material composites to achieve various desired characteristics such as strength, rigidity, elasticity, compliance, biomechanical performance, durability and radiolucency or imaging preference. The components of the bone fastener may also be fabricated from a heterogeneous material such as a combination of two or more of the above-described materials.

It is envisioned that the components of the bone fastener can be manufactured via various methods. For example, bone fastener 05 can be manufactured and assembled via injection-molding, insert-molding, overmolding, compression molding, transfer molding, coextrusion, pultrusion, dip-coating, spray-coating, powder-coating, porous-coating, machining, milling from a solid stock material, and their combinations. One skilled in the art, however, will realize that such materials and fabrication methods suitable for assembly and manufacture, in accordance with the present disclosure, would be appropriate.

Bone fastener 05 can be employed alone or with other hardware, for example, such as, a vertebral rod system or plates, which are configured for attachment to bone during surgical treatment of a spinal disorder.

Radiomarkers may be included in the coating of the outer and/or inner surfaces of the bone fastener 05 for identification under x-ray, fluoroscopy, CT or other imaging techniques. Metallic or ceramic radiomarkers, such as tantalum beads, tantalum pins, titanium pins, titanium endcaps and platinum wires can be used as part of the bone fastener 05 or in conjunction with the bone fastener 05.

In assembly, operation and use, the bone fastener 05 is employed with a surgical procedure for treatment of a spinal disorder affecting a section of a spine of a patient, as discussed herein, for example the bone fastener 05 may be employed into pedicle as part of a surgical procedure to correct spinal fractures, disorders or injury. That is, bone fastener 05 is used as part of surgical procedures for treatment of a condition or injury of an affected section of the spine including, for example one or more vertebrae. It is contemplated that the bone fastener 05 can be used in conjunction with a vertebral rod system, for example bone fastener 05 can be attached to vertebrae V for fusion and/or dynamic stabilization applications of the affected section of the spine so as to facilitate healing and therapeutic treatment, while providing flexion, extension and/or torsion capability.

In use, to treat the affected section of the spine, a medical practitioner obtains access to a surgical site in any appropriate manner, such as through incision and retraction of tissues. It is envisioned that the bone fastener 05 may be used in any existing surgical method or technique including open surgery, mini-open surgery, minimally invasive surgery and percutaneous surgical implantation, whereby vertebrae V is accessed through a micro-incision, or sleeve that provides a protected passageway to the area. Once access to the surgical site is obtained, the particular surgical procedure is performed for treating the spinal disorder. The vertebral rod system including bone fastener 05 is then employed to augment the surgical treatment. As stated herein, the bone fastener 05 can be delivered or implanted in connection with, for example a rod assemble as a pre-assembled device or can be assembled in situ.

## Claims

1. A fenestrated bone fastener (05) comprising:
a head (10);
an elongated shank (15) defining a longitudinal axis having a proximal end (20) and a distal end (30) wherein the proximal end (20) is connected to the head (10) and the elongated shank (15) comprises an inner surface defining a longitudinal cavity (35) disposed along the longitudinal axis and at least one transverse cavity (25) positioned in the elongated shank (15), the at least one transverse cavity (25) is in communication with the longitudinal cavity (35) wherein at least the inner surface defining the transverse cavity (25) has bone conductive material disposed thereon so as to promote bone growth into the transverse cavities (25) to secure the bone fastener (05) to bone, wherein the distal end (30) further comprises cutting features configured to cut into bone as the bone fastener (05) is inserted into bone so as to self-pack the longitudinal cavity (35) with autograft material, **characterized in that** the cutting features are configured as a plurality of blades (60) disposed along the inner surface defining the longitudinal cavity (35), the blades (60) having a cutting edge that spirals along at least a portion of the inner surface of the longitudinal cavity (35).

2. A fenestrated bone fastener (05) according to Claim 1, wherein the inner surface defining the longitudinal cavity (35) comprises bone conductive material disposed thereon so as to promote bone growth into and about the longitudinal cavity (35) so as to secure the bone fastener (05) to bone.

3. A fenestrated bone fastener (05) according to either Claim 1 or Claim 2, wherein the outer surface defining the elongated shank (15) further comprises bone conductive material disposed thereon so as to promote bone growth about the outer surface of the elongated shank (15) and further secure the bone fastener (05) to bone.

4. A fenestrated bone fastener (05) according to any of claims 1-3, wherein the inner surface defining the plurality of traverse cavities (25), the longitudinal cavity (35) and the outer surface of the elongated shank (15) comprises bone conductive material disposed thereon so as to promote bone growth about the outer and inner surfaces of the elongated shank (15) so as to secure the bone fastener (05) to bone.

5. A fenestrated bone fastener (05) according to any of claims 1-3, wherein the elongated shank (15) defines a thickness of the bone fastener (05) and at least one traverse cavity (25) deposed therein extends completely through the thickness of the bone fastener (05).

6. A fenestrated bone fastener (05) according to Claim 5, wherein at least one traverse cavity (25) deposed therein extends partially through the thickness of the bone fastener (05) so as to be continuous with the outside surface of only one side of the bone fastener (05).

7. A fenestrated bone fastener (05) according to any of claims 1-6, wherein at least two of the traverse cavities (25) are disposed at different angular orientations in relationship to each other.

8. A fenestrated bone fastener (05) according to any of claims 1-7, wherein at least one of the traverse cavities (25) are perpendicular to the longitudinal axis of the elongated shank (15).

9. A fenestrated bone fastener (05) according to any of claims 1-8, wherein the bone conductive material is sprayed, layered, fused, coated or textured in a manner or with material that facilitates the growth and attachment of bone.

10. A fenestrated bone fastener (05) according to claim 1, wherein the bone conductive material disposed on the inner surface of the at least one transverse cavity (25) positioned in the elongated shank (15) is selected from the group consisting of bone graft, therapeutic polynucleotides or polypeptides, rigid polymers, biocompatible metals, such as titanium elements, metal powders of titanium or titanium compositions, sterile bone materials, such as allograft or xenograft materials, synthetic bone materials such as coral and calcium compositions, such as hyaluronic acid (HA), calcium phosphate, calcium sulfite, biologically active agents, such as, Bone Morphogenetic Proteins (BMP), Growth and Differentiation Factors Proteins (GDF), cytokines, allogenic demineralized bone, synthetic polymers and copolymers, synthetic copolymers of polyglicolic and polylactic acid, purified collagen, epidermal growth factor (EGF) platelet derived growth factor (PDGF), fibroblast growth factors (FGFs), parathyroid hormone related peptide (PTHrp), insulin-like growth factors (IGFs) and transforming growth factor-beta (TGF-B).

11. A fenestrated bone fastener (05) according to claim 1, wherein the bone conductive material disposed on the inner surface of the at least one transverse cavity (25) positioned in the elongated shank (15) is hyaluronic acid (HA) or Bone Morphogenetic Proteins (BMP).

12. A fenestrated bone fastener (05) according to claim 4, wherein the bone conductive material disposed on the inner surface defining the at least one transverse cavity (25) positioned in the elongated shank (15) and the outer surface of the elongated shank (15) is selected from the group consisting of bone graft, therapeutic polynucleotides or polypeptides, rigid polymers, biocompatible metals, such as titanium elements, metal powders of titanium or titanium compositions, sterile bone materials, such as allograft or xenograft materials, synthetic bone materials such as coral and calcium compositions, such as hyaluronic acid (HA), calcium phosphate, calcium sulfite, biologically active agents, such as, Bone Morphogenetic Proteins (BMP), Growth and Differentiation Factors Proteins (GDF), cytokines, allogenic demineralized bone, synthetic polymers and copolymers, synthetic copolymers of polyglicolic and polylactic acid, purified collagen, epidermal growth factor (EGF) platelet derived growth factor (PDGF), fibroblast growth factors (FGFs), parathyroid hormone related peptide (PTHrp), insulin-like growth factors (IGFs) and transforming growth factor-beta (TGF-B).

13. A fenestrated bone fastener (05) according to claim 4, wherein the bone conductive material disposed on the inner surface of the at least one transverse cavity (25) positioned in the elongated shank (15) is hyaluronic acid (HA) or Bone Morphogenetic Proteins (BMP).

## Patentansprüche

1. Gefenstertes Knochenbefestigungsmittel (05), aufweisend:
einen Kopf (10),
einen eine Längsachse definierenden länglichen Schaft (15), welcher ein proximales Ende (20) und ein distales Ende (30) aufweist, wobei das proximale Ende (20) mit dem Kopf (10) verbunden ist und der längliche Schaft (15) eine Innenfläche aufweist, die eine entlang der Längsachse angeordnete longitudinale Kavität (35) und wenigstens eine in dem länglichen Schaft (15) angeordnete transversale Kavität (25) definiert, wobei die wenigstens eine transversale Kavität (25) in Verbindung mit der longitudinalen Kavität (35) steht,
wobei wenigstens die die transversale Kavität (25) definierende Innenfläche daran angeordnetes knochenleitfähiges Material aufweist, um Knochenwachstum in die transversalen Kavitäten (25) zu fördern, um das Knochenbefestigungsmittel (05) an einen Knochen zu sichern, wobei das distale Ende (30) ferner Schneidmerkmale aufweist, die dazu eingerichtet sind,
in einen Knochen zu schneiden, wenn das Knochenbefestigungsmittel (05) in einen Knochen eingeführt wird, um die longitudinale Kavität (35) mit Autograftmaterial selbst zu bepacken,
**dadurch gekennzeichnet, dass** die Schneidmerkmale als eine Mehrzahl von Klingen (60) ausgebildet sind, welche entlang der die longitudinale Kavität (35) definierenden Innenfläche angeordnet sind, wobei die Klingen (60) eine Schneidkante aufweisen, die sich spiralförmig entlang wenigstens eines Abschnitts der Innenfläche der longitudinalen Kavität (35) erstreckt.

2. Gefenstertes Knochenbefestigungsmittel (05) nach Anspruch 1,
wobei die die longitudinale Kavität (35) definierende Innenfläche daran angeordnetes knochenleitfähiges Material aufweist, um Knochenwachstum in die und um die longitudinale Kavität (35) herum zu fördern, um das Knochenbefestigungsmittel (05) an einen Knochen zu sichern.

3. Gefenstertes Knochenbefestigungsmittel (05) nach Anspruch 1 oder Anspruch 2,
wobei die den länglichen Schaft (15) definierende Außenfläche ferner daran angeordnetes knochenleitfähiges Material aufweist, um Knochenwachstum um die Außenfläche des länglichen Schafts (15) herum zu fördern und um das Knochenbefestigungsmittel (05) weiter an einen Knochen zu sichern.

4. Gefenstertes Knochenbefestigungsmittel (05) nach einem der Ansprüche 1 bis 3,
wobei die die Mehrzahl von transversalen Kavitäten (25) definierende Innenfläche, die longitudinale Kavität (35) und die Außenfläche des länglichen Schafts (15) daran angeordnetes knochenleitfähiges Material aufweisen, um Knochenwachstum um die Außen- und die Innenfläche des länglichen Schafts (15) herum zu fördern, um das Knochenbefestigungsmittel (05) an einen Knochen zu sichern.

5. Gefenstertes Knochenbefestigungsmittel (05) nach einem der Ansprüche 1 bis 3,
wobei der längliche Schaft (15) eine Dicke des Knochenbefestigungsmittels (05) definiert und wobei sich wenigstens eine darin angeordnete transversale Kavität (25) vollständig durch die Dicke des Knochenbefestigungsmittels (05) erstreckt.

6. Gefenstertes Knochenbefestigungsmittel (05) nach Anspruch 5,
wobei sich wenigstens eine darin angeordnete transversale Kavität (25) teilweise durch die Dicke des Knochenbefestigungsmittels (05) erstreckt, um durchgehend mit der Außenfläche nur einer Seite des Knochenbefestigungsmittels (05) zu sein.

7. Gefenstertes Knochenbefestigungsmittel (05) nach einem der Ansprüche 1 bis 6,
wobei wenigstens zwei der transversalen Kavitäten (25) mit voneinander verschiedenen Winkelorientierungen angeordnet sind.

8. Gefenstertes Knochenbefestigungsmittel (05) nach einem der Ansprüche 1 bis 7,
wobei wenigstens eine der transversalen Kavitäten (25) orthogonal zu der Längsachse des länglichen Schafts (15) ist.

9. Gefenstertes Knochenbefestigungsmittel (05) nach einem der Ansprüche 1 bis 8,
wobei das knochenleitfähige Material auf eine Weise oder mit einem Material aufgesprüht, geschichtet, aufgeschmolzen, beschichtet oder strukturiert ist, welche bzw. welches das Wachstum und die Anbringung von Knochen erleichtert.

10. Gefenstertes Knochenbefestigungsmittel (05) nach Anspruch 1,
wobei das an der Innenfläche der wenigstens einen in dem länglichen Schaft (15) angeordneten transversalen Kavität (25) angeordnete knochenleitfähige Material aus der Gruppe ausgewählt ist, bestehend aus: Knochentransplantat, therapeutischen Polynucleotiden oder Polypeptiden, harten Polymeren, biokompatiblen Metallen, wie etwa Titanelementen, Metallpulvern aus Titan oder Titanzusammensetzungen, sterilen Knochenmaterialien, wie etwa Allograft- oder Xenograftmaterialien, synthetischen Knochenmaterialien, wie etwa Korallen- und Kalziumzusammensetzungen, wie etwa Hyaluronsäure (HA), Kalziumphosphat, Kalziumsulfit, biologisch aktiven Mitteln, wie etwa knochenmorphogenetischen Proteinen (BMP), Wachstums- und Differenzierungsfaktoren-Proteinen (GDF), Zytokinen, allogen demineralisierten Knochen, synthetischen Polymeren und Copolymeren, synthetischen Copolymeren aus Polyglycolsäure und Polymilchsäure, gereinigtem Kollagen, dem epidermalen Wachstumsfaktor (EGF), einem Platelet-Derived Wachstumsfaktor (PDGF), Fibroblasten-Wachstumsfaktoren (FGFs), Parathyroid-Hormone-Related-Peptiden (PTHrp), insulinählichen Wachstumsfaktoren (IGFs) und dem transformierenden Wachstumsfaktor beta (TGF-B).

11. Gefenstertes Knochenbefestigungsmittel (05) nach Anspruch 1,
wobei das an der Innenfläche der wenigstens einen in dem länglichen Schaft (15) angeordneten transversalen Kavität (25) angeordnete knochenleitfähige Material Hyaluronsäure (HA) oder knochenmorphogenetische Proteine (BMP) ist.

12. Gefenstertes Knochenbefestigungsmittel (05) nach Anspruch 4,
wobei das knochenleitfähige Material, welches an der die wenigstens eine in dem länglichen Schaft (15) angeordnete transversale Kavität (25) definierenden Innenfläche und an der Außenfläche des länglichen Schafts (15) angeordnet ist, ausgewählt ist aus der Gruppe, bestehend aus: Knochentransplantat, therapeutischen Polynucleotiden oder Polypeptiden, harten Polymeren, biokompatiblen Metallen, wie etwa Titanelementen, Metallpulvern aus Titan oder Titanzusammensetzungen, sterilen Knochenmaterialien, wie etwa Allograft- oder Xenograftmaterialien, synthetischen Knochenmaterialien, wie etwa Korallen- und Kalziumzusammensetzungen, wie etwa Hyaluronsäure (HA), Kalziumphosphat, Kalziumsulfit, biologisch aktiven Mitteln, wie etwa knochenmorphogenetischen Proteinen (BMP), Wachstums- und Differenzierungsfaktoren-Proteinen (GDF), Zytokinen, allogen demineralisierten Knochen, synthetischen Polymeren und Copolymeren, synthetischen Copolymeren aus Polyglycolsäure und Polymilchsäure, gereinigtem Kollagen, dem epidermalen Wachstumsfaktor (EGF), einem Platelet-Derived Wachstumsfaktor (PDGF), Fibroblasten-Wachstumsfaktoren (FGFs), Parathyroid-Hormone-Related-Peptiden (PTHrp), insulinählichen Wachstumsfaktoren (IGFs) und dem transformierenden Wachstumsfaktor beta (TGF-B).

13. Gefenstertes Knochenbefestigungsmittel (05) nach Anspruch 4,
wobei das an der Innenfläche der wenigstens einen in dem länglichen Schaft (15) angeordneten transversalen Kavität (25) angeordnete knochenleitfähige Material Hyaluronsäure (HA) oder knochenmorphogenetische Proteine (BMP) ist.

## Revendications

1. Elément de fixation d'os fenêtré (05) comprenant :
une tête (10) ;
une tige allongée (15) définissant un axe longitudinal ayant une extrémité proximale (20) et une extrémité distale (30), l'extrémité proximale (20) étant connectée à la tête (10) et la tige allongée (15) comprenant une surface intérieure définissant une cavité longitudinale (35) disposée le long de l'axe longitudinal et au moins une cavité transversale (25) positionnée dans la tige allongée (15), l'au moins une cavité transversale (25) étant en communication avec la cavité longitudinale (35), au moins la surface intérieure qui définit la cavité transversale (25) ayant un matériau conducteur osseux disposé dessus de façon à favoriser la croissance osseuse dans les cavités transversales (25) afin d'assujettir l'élément de fixation d'os (05) à un os, l'extrémité distale (30) comprenant en outre des caractéristiques de découpe configurées pour couper dans l'os lorsque l'élément de fixation d'os (05) est inséré dans un os de façon à remplir automatiquement la cavité longitudinale (35) avec un matériau d'autogreffe,
**caractérisé en ce que** les caractéristiques de découpe sont configurées sous la forme d'une pluralité de lames (60) disposées le long de la surface intérieure définissant la cavité longitudinale (35), les lames (60) ayant un bord de coupe qui forme une spirale le long d'au moins une partie de la surface intérieure de la cavité longitudinale (35).

2. Elément de fixation d'os fenêtré (05) selon la revendication 1, dans lequel la surface intérieure définissant la cavité longitudinale (35) comprend un matériau conducteur osseux disposé dessus de façon à favoriser la croissance osseuse dans et autour de la cavité longitudinale (35) et de façon à assujettir l'élément de fixation d'os (05) à un os.

3. Elément de fixation d'os fenêtré (05) selon l'une ou l'autre des revendications 1 et 2, dans lequel la surface extérieure définissant la tige allongée (15) comprend en outre un matériau conducteur osseux disposé dessus de façon à favoriser la croissance osseuse autour de la surface extérieure de la tige allongée (15) et en outre assujettir l'élément de fixation d'os (05) à un os.

4. Elément de fixation d'os fenêtré (05) selon l'une quelconque des revendications 1 à 3, dans lequel la surface intérieure définissant la pluralité de cavités transversales (25), la cavité longitudinale (35) et la surface extérieure de la tige allongée (15) comprend un matériau conducteur osseux disposé dessus de façon à favoriser la croissance osseuse autour des surfaces extérieure et intérieure de la tige allongée (15) afin d'assujettir l'élément de fixation d'os (05) à un os.

5. Elément de fixation d'os fenêtré (05) selon l'une quelconque des revendications 1 à 3, dans lequel la tige allongée (15) définit une épaisseur de l'élément de fixation d'os (05) et au moins une cavité transversale (25) déposée dedans s'étend complètement à travers l'épaisseur de l'élément de fixation d'os (05).

6. Elément de fixation d'os fenêtré (05) selon la revendication 5, dans lequel au moins une cavité transversale (25) déposée dedans s'étend partiellement à travers l'épaisseur de l'élément de fixation d'os (05) de façon à être continue avec la surface extérieure de seulement un côté de l'élément de fixation d'os (05).

7. Elément de fixation d'os fenêtré (05) selon l'une quelconque des revendications 1 à 6, dans lequel au moins deux des cavités transversales (25) sont disposées selon des orientations angulaires différentes l'une par rapport à l'autre.

8. Elément de fixation d'os fenêtré (05) selon l'une quelconque des revendications 1 à 7, dans lequel au moins l'une des cavités transversales (25) est perpendiculaire à l'axe longitudinal de la tige allongée (15).

9. Elément de fixation d'os fenêtré (05) selon l'une quelconque des revendications 1 à 8, dans lequel le matériau conducteur osseux est pulvérisé, stratifié, fusionné, déposé sous forme de revêtement ou texturé d'une manière ou avec un matériau qui facilite la croissance et l'attachement osseux.

10. Elément de fixation d'os fenêtré (05) selon la revendication 1, dans lequel le matériau conducteur osseux disposé sur la surface intérieure de l'au moins une cavité transversale (25) positionnée dans la tige allongée (15) est choisi dans le groupe constitué par une greffe d'os, les polynucléotides ou polypeptides thérapeutiques, les polymères rigides, les métaux biocompatibles, tels que les éléments en titane, les poudres métalliques de titane ou de compositions de titane, les matériaux osseux stériles, tels que les matériaux d'allogreffe ou de xénogreffe, les matériaux osseux synthétiques tels que les compositions de corail et de calcium, comme l'acide hyaluronique (HA), le phosphate de calcium, le sulfite de calcium, les agents biologiquement actifs, tels que les protéines morphogénétiques osseuses (BMP), les protéines de facteurs de croissance et de différenciation (GDF), les cytokines, l'os déminéralisé allogénique, les polymères et copolymères synthétiques, les copolymères synthétiques de poly(acide glycolique) et de poly(acide lactique), le collagène purifié, le facteur de croissance épidermique (EGF), le facteur de croissance dérivé des plaquettes (PDGF), les facteurs de croissance des fibroblastes (FGF), le peptide apparenté à la parathormone (PTHrp), les facteurs de croissance insulinomimétiques (IGF) et le facteur de croissance transformant bêta (TGF-B).

11. Elément de fixation d'os fenêtré (05) selon la revendication 1, dans lequel le matériau conducteur osseux disposé sur la surface intérieure de l'au moins une cavité transversale (25) positionnée dans la tige allongée (15) est l'acide hyaluronique (HA) ou des protéines morphogénétiques osseuses (BMP).

12. Elément de fixation d'os fenêtré (05) selon la revendication 4, dans lequel le matériau conducteur osseux disposé sur la surface intérieure définissant l'au moins une cavité transversale (25) positionnée dans la tige allongée (15) et la surface supérieure de la tige allongée (15) est choisi dans le groupe constitué par une greffe d'os, les polynucléotides ou polypeptides thérapeutiques, les polymères rigides, les métaux biocompatibles, tels que les éléments en titane, les poudres métalliques de titane ou de compositions de titane, les matériaux osseux stériles, tels que les matériaux d'allogreffe ou de xénogreffe, les matériaux osseux synthétiques tels que les compositions de corail et de calcium, comme l'acide hyaluronique (HA), le phosphate de calcium, le sulfite de calcium, les agents biologiquement actifs, tels que les protéines morphogénétiques osseuses (BMP), les protéines de facteurs de croissance et de différenciation (GDF), les cytokines, l'os déminéralisé allogénique, les polymères et copolymères synthétiques, les copolymères synthétiques de poly(acide glycolique) et de poly(acide lactique), le collagène purifié, le facteur de croissance épidermique (EGF), le facteur de croissance dérivé des plaquettes (PDGF), les facteurs de croissance des fibroblastes (FGF), le peptide apparenté à la parathormone (PTHrp), les facteurs de croissance insulinomimétiques (IGF) et le facteur de croissance transformant bêta (TGF-B).

13. Elément de fixation d'os fenêtré (05) selon la revendication 4, dans lequel le matériau conducteur osseux disposé sur la surface intérieure de l'au moins une cavité transversale (25) positionnée dans la tige allongée (15) est l'acide hyaluronique (HA) ou des protéines morphogénétiques osseuses (BMP).
